# EUROPÄISCHE PATENTSCHRIFT

(11) **EP 0 879 057 B1**
(45) Veröffentlichungstag und Bekanntmachung des Hinweises auf die Patenterteilung: **05.06.2002**
(21) Anmeldenummer: 97902208.4
(22) Anmeldetag: 23.01.1997
(51) Int. Cl.: A61K 31/53, A61K 9/16

(54) **GRANULATE VON TRIAZINEN**
GRANULES FROM TRIAZINES
GRANULES DE TRIAZINES

(30) Priorität: 05.02.1996 DE 19603984
(43) Veröffentlichungstag der Anmeldung: 25.11.1998
(73) Patentinhaber: BAYER AG, 51368 Leverkusen (DE)
(72) Erfinder: GROFMEYER, Dorothea, D-40764 Langenfeld (DE); MUNDT, Christian, D-40699 Erkrath (DE); WEGNER, Christian, D-51061 Köln (DE)
(86) Internationale Anmeldenummer: EP9700310
(87) Internationale Veröffentlichungsnummer: WO9728804

(56) Entgegenhaltungen:
- EP-A- 0 279 218
- WO-A-94/21260
- WO-A-96/17611

## Beschreibung

Die vorliegende Erfindung betrifft Granulate von Triazinen mit Ausnahme von Lamotrigine sowie ihre Herstellung.

Toltrazuril (1-Methyl-3-(3-methyl-4-(4-(trifluormethyl)thio)phenoxy)phenyl)-1,3,5-triazin-2,4,6(1H, 3H, 5H)trion) ist ein bekannter Wirkstoff gegen Coccidien. Er wird üblicherweise angewendet in Form von wäßrigen Lösungen oder Suspensionen. Für viele Anwendungsgebiete lassen sich diese Lösungen nur schwer einsetzen.

Die vorliegende Erfindung betrifft Granulate von Triazinen mit Ausnahme von Lamotigine folgender Zusammensetzung
a) Wirkstoff 0,5 bis 10 % (Gewicht)
b) Stärke 10 bis 40 % (Gewicht)
c) Lactose 30 bis 60 % (Gewicht)
d) Polyvinylpyrrolidon 5 bis 35 % (Gewicht)
mit einer mittleren Korngrößenverteilung zwischen 40 bis 400 µm und einer Obergrenze der Korngröße von 2 000 µm.

Die Herstellung des Granulats erfolgt, indem man die Komponenten a) bis d) in einem geeigneten Misch- oder Rührgefäß vormischt und mit einer Lösung von Polyvinylpyrrolidon besprüht. Anschließend wird das Granulat getrocknet und gegebenenfalls abgesiebt.

Man erhält ein staubfreies, trockenes, leicht fließendes und leicht dosierbares Granulat. Bevorzugt wird dieses Granulat zur Behandlung von Coccidioseerkrankungen von Katzen und anderen Heim- und Hobbytieren eingesetzt. Neben Katzen zählen zu den Heim- und Hobbytieren Hunde, Kaninchen, Ziervögel.

Die Anwendung des Granulats erfolgt durch Vermischen mit festem, halbfestem oder flüssigem Futter. Futter das mit dem erfindungsgemäßen Granulat versetzt ist wird erfahrungsgemäß ohne Probleme auch von empfindlichen Tieren aufgenommen.

Als Wirkstoffe seien genannt symmetrische 1,3,5-Triazin-2,4,6(1H, 3H, 5H)-trione wie Toltrazuril, unsymmetrische 1,2,4-Triazin-3,5-dione wie Diclazuril (2,6-Dichlor-α-(4-chlorphenyl)-4-(4,5-dihydro-3,5-dioxo-1,2,4-triazin-2(3H)-yl)-phenylacetonitril).

Bevorzugt genannt sei Toltrazuril. Die Wirkstoffe werden mit 0,5 bis 10, bevorzugt 1 bis 5, besonders bevorzugt 2 bis 4 Gew.-% eingesetzt.

Als Stärke seien die üblichen Stärkearten wie Kartoffelstärke, Maisstärke, Getreidestärke genannt. Bevorzugt sei Maisstärke genannt. Stärke wird mit 10 bis 40, bevorzugt 20 bis 30, besonders bevorzugt um ca. 25 Gew.-% eingesetzt.

Lactose wird als Monohydrat eingesetzt in Konzentrationen von 30 bis 60, bevorzugt 50 bis 60, besonders bevorzugt um ca. 60 Gew.-%.

Polyvinylpyrrolidon (PVP) wird bevorzugt als mittelmolekulares PVP mit einem K-Wert von 24 bis 32 eingesetzt. Besonders bevorzugt ist PVP mit einem K-Wert von 24 bis 30, besonders bevorzugt von 25. Als Lösungsmittel für PVP wird bevorzugt Wasser eingesetzt. Als Lösungsmittel seien auch organische Lösungsmittel allein oder in Mischung mit Wasser genannt.

Ein K-Wert von 25 entspricht einem Gewichtsmittel der Molekülmasse von 28 000 bis 34 000. Der K-Wert charakterisiert die mittlere Molekülmasse.

PVP wird bevorzugt in einer Gesamtmenge von 10 bis 30 Gew.-% eingesetzt.

Die Korngrößenverteilung liegt zwischen 40 bis 400 µm, bevorzugt zwischen 80 und 200 µm, mit einer Obergrenze bei 2 000 µm, bevorzugt bei 1 000 µm.

Die Herstellung der erfindungsgemäßen Granulate erfolgt in Feststoffmischern, bevorzugt in Wirbelschichtgranulatoren.

Zur Herstellung werden die Einzelkomponenten bei Raumtemperatur homogen gemischt. Danach wird die Mischung mit einer 10 bis 50 %igen, bevorzugt 30 bis 40 %igen, besonders bevorzugt einer 30 %igen, wäßrigen PVP-Lösung besprüht und gegebenenfalls mit weiterem Wasser besprüht. Pro kg Mischung wird 100 bis 300, bevorzugt 150 bis 200 ml Sprühlösung eingesetzt. Die Granulierung erfolgt bei 20 bis 50°C, bevorzugt bei 25 bis 40°C.

Das Granulat wird anschließend bei ca. 40 bis 70°C, bevorzugt bei 50 bis 60°C getrocknet.

### Beispiel 1

### Herstellung:

1. 500 g Polyvinylpyrrolidon mit K-Wert 25 = PVP-25 werden bei Raumtemperatur innerhalb von 2 Stunden unter Rühren mit einem Zahnscheibenrührer in 1250 g Wasser (entmineralisiert) eingetragen. Es wird 1 Stunde bis zum vollständigen Lösen nachgerührt.
2. 2500 g Maisstärke, 1000 g PVP-25, 200 g Toltrazuril und 5800 g Milchzucker werden im Wirbelschichtgranulator vom Typ Aeromatic S 2 vorgelegt. Man homogenisiert 10 Minuten im Wirbelbett unter Fluidisieren mit 150 bis 200 m³/h und Temperierung auf 25 bis 30°C.
3. Die Vorwärmphase bis zum Erreichen von 25 bis 40°C Zulufttemperatur soll 2 bis 4 Minuten betragen.
4. Im Wirbelschichtgranulator wird die Vormischung bei 35 bis 40°C Zuluft, 22 bis 30°C Abluft und einer Zuluftmenge von 150 bis 200 m³/h unter Aufsprühen von 30 g Sprühlösung/Minute bei 2 bar Sprühdruck granuliert.
   Gesprüht wird die PVP-Vorlösung aus Herstellung 1.
   Zum Nachspülen der Düse werden etwa 100 g Wasser denat. nachgesetzt.
   Anschließend wird bei 55°C Zuluft bis 42-45°C Ablufttemperatur mit 150-200 m³/h getrocknet.
5. Um evtl. vorhandenes Grobgranulat oder Wandanbackungen auszuschleusen wird über 2000 um abgesiebt.

## Patentansprüche

1. Granulate von Triazinen mit Ausnahme von Lamotrigine, folgender Zusammensetzung
a) Wirkstoff 0,5 bis 10 % (Gewicht)
b) Stärke 10 bis 40 % (Gewicht)
c). Lactose 30 bis 60 % (Gewicht)
d) Polyvinylpyrrolidon 5 bis 35 % (Gewicht)
mit einer mittleren Korngrößenverteilung zwischen 40 bis 400 µm und einer Obergrenze der Korngröße bei 2 000 µm.

2. Verfahren zur Herstellung von Granulaten von Triazinen mit Ausnahme von Lamotrigine, folgender Zusammensetzung
a) Wirkstoff 0,5 bis 10 % (Gewicht)
b) Stärke 10 bis 40 % (Gewicht)
c) Lactose 30 bis 60 % (Gewicht)
d) Polyvinylpyrrolidon 5 bis 35 % (Gewicht)
mit einer Korngrößenverteilung zwischen 40 bis 400 µm und einer Obergrenze der Korngröße bei 2 000 µm, **dadurch gekennzeichnet, daß** man die Komponenten a) bis d) in einem geeigneten Misch- oder Rührgefäß vormischt und mit einer Lösung von Polyvinylpyrrolidon besprüht, trocknet und gegebenenfalls absiebt.

## Claims

1. Granules of triazines, with the exception of lamotrigine, of the following composition
a) active compound 0.5 to 10% (weight)
b) starch 10 to 40% (weight)
c) lactose 30 to 60% (weight)
d) polyvinylpyrrolidone 5 to 35% (weight)
having an average particle size distribution of between 40 to 400 µm and an upper limit of the particle size at 2 000 µm.

2. Process for the preparation of granules of triazines, with the exception of lamotrigine, of the following composition
a) active compound 0.5 to 10% (weight)
b) starch 10 to 40% (weight)
c) lactose 30 to 60% (weight)
d) polyvinylpyrrolidone 5 to 35% (weight)
with a particle size distribution of between 40 to 400 µm and an upper limit of the particle size at 2 000 µm, **characterized in that** the components a) to d) are premixed in a suitable mixing or stirring vessel and sprayed with a solution of polyvinylpyrrolidone, dried and, if necessary, screened off.

## Revendications

1. Granulés de triazines, à l'exception de la lamotrigine, de composition suivante :
a) Substance active, 0,5 à 10 % (en poids)
b) Amidon, 10 à 40 % (en poids)
c) Lactose, 30 à 60 % (en poids)
d) Polyvinylpyrrolidone, 5 à 35 % (en poids)
avec une répartition granulométrique moyenne entre 40 et 400 µm et une limite supérieure de diamètre des grains de 2000 µm.

2. Procédé de production de granulés de triazines à l'exception de la lamotrigine, de composition suivante :
a) Substance active, 0,5 à 10 % (en poids)
b) Amidon, 10 à 40 % (en poids)
c) Lactose, 30 à 60 % (en poids)
d) Polyvinylpyrrolidone, 5 à 35 % (en poids) avec une répartition granulométrique entre 40 et 400 µm
et une limite supérieure de diamètre des grains de 2000 µm, **caractérisé en ce qu'**on mélange tout d'abord les composants a) à b) dans un récipient mélangeur ou agitateur approprié et on pulvérise sur le mélange une solution de polyvinylpyrrolidone, on sèche le mélange et on le tamise le cas échéant.
